# EUROPEAN PATENT SPECIFICATION

(11) **EP 2 395 912 B1**
(45) Date of publication and mention of the grant of the patent: **17.04.2013**
(21) Application number: 10704846.4
(22) Date of filing: 01.02.2010
(51) Int. Cl.: A61B 5/05

(54) **METHOD AND DEVICE FOR MAGNETIC INDUCTION TOMOGRAPHY**
VERFAHREN UND VORRICHTUNG FÜR DIE MAGNETINDUKTIONSTOMOGRAPHIE
PROCEDE ET DISPOSITIF DE TOMOGRAPHIE PAR INDUCTION MAGNETIQUE

(30) Priority: 13.02.2009 CN 200910007407
(43) Date of publication of application: 21.12.2011
(73) Proprietor: Koninklijke Philips Electronics N.V., 5621 BA Eindhoven (NL); Philips Intellectual Property & Standards GmbH, 20099 Hamburg (DE)
(72) Inventor: IGNEY, Claudia Hannelore, 52066 Aachen (DE); HAMSCH, Matthias, 52066 Aachen (DE); MAZURKEWITZ, Peter, 200233 Aachen (DE); LUEDEKE, Kai-Michael, 200233 Aachen (DE)
(74) Representative: Van Velzen, Maaike Mathilde
(86) International application number: PCT/IB2010/050422
(87) International publication number: WO 2010/092503

(56) References cited:
- WO-A2-2007/029138
- Z. Z. YU, A. J. PEYTON: "Development of sensor arrays for electromagnetic inductive tomography: compensation of large background signals" TRANS INST MC, vol. 20, no. 4, 1998, pages 195-202, XP009133082
- GRIFFITHS H ET AL: "Magnetic induction tomography: a measuring system for biologicaltissues" ANNALS OF THE NEW YORK ACADEMY OF SCIENCES, NEW YORK ACADEMY OF SCIENCES, NEW YORK, NY, US LNKD- DOI:10.1111/J.1749-6632.1999.TB09481.X, vol. 873, 1 April 1999 (1999-04-01), pages 335-345, XP009133070 ISSN: 0077-8923

## Description

### FIELD OF THE INVENTION

The invention relates to magnetic induction tomography, in particular to a method and a device that reduce the dynamic range of measurement coils in magnetic induction tomography.

### BACKGROUND OF THE INVENTION

Magnetic induction tomography (MIT) is a non-invasive and contactless imaging technique with applications in industry and medical imaging. In contrast to other electrical imaging techniques, MIT does not require direct contact of the sensors with the object of interest for imaging. MIT is used to reconstruct the spatial distribution of the passive electrical properties inside the object of interest, for example, conductivity σ.

Prior art patent application WO2007072343 discloses a magnetic induction tomography system for studying the electromagnetic properties of an object. The system comprises: one or more generator coils adapted for generating a primary magnetic field, said primary magnetic field inducing an eddy current in the object; one or more sensor coils adapted for sensing a secondary magnetic field, said secondary magnetic field being generated as a result of said eddy current, and means for providing a relative movement between one or more generator coils and/or one or more sensor coils, on the one hand, and the object to be studied, on the other hand.

There are many technical challenges that must be overcome in order to precisely measure the electromagnetic properties of the object. In an MIT system, an alternating current through a transmitting coil produces an alternating primary magnetic field. When this magnetic field passes through a conducting material, eddy currents are induced. These eddy currents produce a secondary alternating magnetic field, which is much smaller than the primary magnetic field. The intended reconstruction of the measurement is performed with a measurement coil based on the induced voltage by the secondary magnetic filed. Unfortunately, the much larger primary magnetic field is present simultaneously and induces proportional voltages, which are much higher. Consequently, the total induced voltage has to be measured with extremely high precision (in magnitude and phase) to be able to extract the small secondary magnetic field-related voltages with sufficient accuracy. This is a first aspect of the measurement challenge produced by the presence of the strong primary magnetic field. The paper 'Development of sensor arrays for electromagnetic inductive tomography: compensation of large background signal values' in Trans.Inst. MC 20(1998)195-202 by Z.Z. Yu and A.J. Peyton discloses a device and method according to the preamble of claims 1 and 5 and mentions to reduce the background signal by positioning the excitation and detection coils in positions of zero mutual coupling.

When the magnetic induction tomography system comprises multiple transmitting and measurement coils, because of the large spatial variation of the primary magnetic field between the measurement coils next to the transmitting coils (being excited) and those farthest away, a measurement system with a very high dynamic range is required. This is a second aspect of the measurement challenge.

### SUMMARY OF THE INVENTION

An object of this invention is to provide an improved coil arrangement for magnetic induction tomography.

According to of the invention, it provides a device for reconstructing images of an object of interest, comprising:
- a plurality of transmitting coils for generating a primary magnetic field;
- a plurality of measurement coils; and
- means for selecting and exciting a first pair of transmitting coils among the plurality of transmitting coils,
wherein the first pair of transmitting coils are selected and excited in a way such that the primary magnetic field generated by the first pair of transmitting coils is minimized at the location of the at least one measurement coil among the plurality of measurement coils.

By minimizing the primary magnetic field at the location of measurement coil(s), the device can reduce the dynamic range of measurement coils, resulting in simplified hardware design for magnetic induction tomography systems.

According to the invention, the means is further arranged to select and excite a second pair of transmitting coils among the plurality of transmitting coils, and the second pair of transmitting coils is selected and excited in such a way that the primary magnetic field generated by the second pair of transmitting coils is minimized at the location of the at least one first measurement coil.

By selecting and exciting multiple pairs of transmitting coils in sequence or in combination at the same time, a set of measurement data can be obtained by the same measurement coil(s) for further image reconstruction.

In another embodiment, the first or second pair of transmitting coils is excited by simultaneously providing each coil with alternating current of equal magnitude and opposite direction. In this way, the primary magnetic field generated by the first and/or second pair of transmitting coils is zero in theory at the location of the at least one first measurement coil, resulting in improved precision of measurement.

In a further embodiment, the plurality of transmitting coils and the plurality of measurement coils are arranged in an annular array. The advantage of this arrangement is that the pair of transmitting coils is easy to be selected based on the symmetry of the transmitting coils.

According to a second aspect of the invention, it provides a method of reconstructing images of an object of interest, the method comprising the steps of:
- selecting a first pair of transmitting coils among the plurality of transmitting coils;
- generating a primary magnetic field to be applied to the object of interest by the first pair of transmitting coils; and
- measuring electrical signals induced by a secondary magnetic field for image reconstruction by at least one measurement coil, the secondary magnetic field being generated by the object of interest in response to the primary magnetic field,
wherein the first pair of transmitting coils is selected and excited in a way such that the primary magnetic field generated by the first pair of transmitting coils is minimized at the location of the at least one measurement coil. The method of the invention further comprises a step of selecting and exciting a second pair of transmitting coils among the plurality of transmitting coils, wherein the second pair of transmitting coils is selected and excited in such a way that the primary magnetic field generated by the second pair of transmitting coils is minimized at the location of the at least one first measurement coil. Detailed explanations and other aspects of the invention are given below.

### DESCRIPTION OF THE DRAWINGS

The above and other objects and features of the present invention will become more apparent from the following detailed description considered in connection with the accompanying drawings, in which:
Fig.1 shows an embodiment of the device in accordance with the invention.
Fig.2 shows a schematic view of a primary magnetic field in accordance with the invention.
Fig.3 is a flowchart of the method in accordance with the invention.
The same reference numerals are used to denote similar parts throughout the Figures.

### DETAILED DESCRIPTION

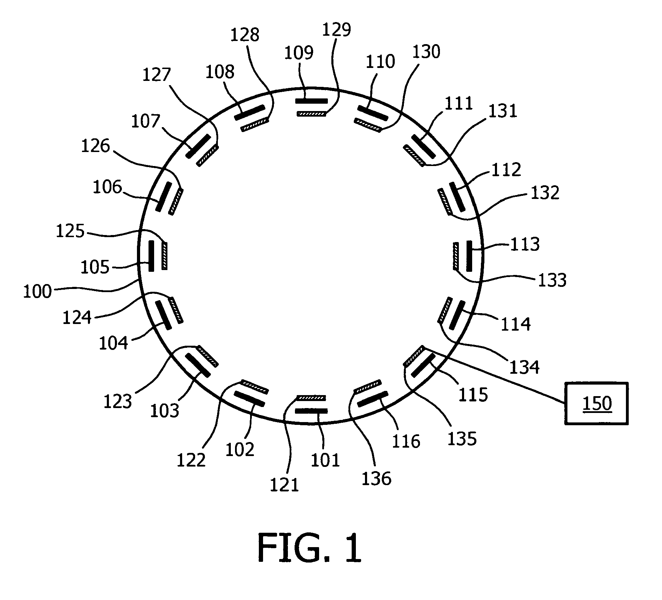

Fig.1 shows an embodiment of the device in accordance with the invention.

Referring to Fig. 1, the device reconstructing images of an object of interest comprises a plurality of transmitting coils 102, 103, 115, 116 for generating a primary magnetic field.

The device further comprises a plurality of measurement coils 121, 129 for measuring electrical signals induced by a secondary magnetic field for image reconstruction, the secondary magnetic field being generated by the object of interest in response to the primary magnetic field.

In detail, when one or more transmitting coils are provided with alternating current, they generate a primary magnetic field that induces eddy currents in the object of interest. The object of interest (not shown in Fig. 1), which can be any conductive material or human tissue, is usually put in the measurement area formed by the coil arrangement. The eddy current generates a secondary magnetic field that induces electrical signals in the measurement coil.

The device further comprises means 150 for selecting and exciting a first pair of transmitting coils 102, 116 among the plurality of transmitting coils, wherein the first pair of transmitting coils are selected and excited in a way such that the primary magnetic field generated by the first pair of transmitting coils is minimized at the location of the at least one measurement coil 121, 129 among the plurality of measurement coils. In the description of the invention, excitation means serve to switch on and provide alternating current to the transmitting coils for generating a primary magnetic field.

It will be understood that the plurality of transmitting coils and the plurality of measurement coils can be arranged in different arrays.

It is advantageous that the plurality of transmitting coils and the plurality of measurement coils are arranged in an annular array. Particularly, the plurality of transmitting coils and the plurality of measurement coils can be arranged in pairs and situated around the inner wall of a tubular container 100.

An advantage of arranging transmitting coils and measurement coils in an annular array is that the pair of transmitting coil for excitation can be easily selected because of the symmetry of the coils.

In another embodiment, a second pair of transmitting coils, 102, 115, can be selected and excited in a similar way so as to generate a primary magnetic field that is minimized at the location of measurement coils 121 and 129.

It will be understood that the first and second pair of transmitting coils can be excited in sequence or at the same time. Table 1 gives an exemplary coil combination for excitation when the coil arrangement comprises 16 transmitting coils and 16 measuring coils, and the transmitting and measuring coils are arranged in an annular array. In the table, Le1, Le2 denote transmitting coils, Lm1, Lm2 denote measuring coils.

| Le1 | Le2 | Lm1 | Lm2 | Le1 | Le2 | Lm1 | Lm2 | Le1 | Le2 | Lm1 | Lm2 |
|---|---|---|---|---|---|---|---|---|---|---|---|
| 2 | 16 | 1 | 9 | 3 | 1 | 2 | 10 | 4 | 2 | 3 | 11 |
| 3 | 15 | 1 | 9 | 4 | 16 | 2 | 10 | 5 | 1 | 3 | 11 |
| 4 | 14 | 1 | 9 | 5 | 15 | 2 | 10 | 6 | 16 | 3 | 11 |
| 5 | 13 | 1 | 9 | 6 | 14 | 2 | 10 | 7 | 15 | 3 | 11 |
| 6 | 12 | 1 | 9 | 7 | 13 | 2 | 10 | 8 | 14 | 3 | 11 |
| 7 | 11 | 1 | 9 | 8 | 12 | 2 | 10 | 9 | 13 | 3 | 11 |
| 8 | 10 | 1 | 9 | 9 | 11 | 2 | 10 | 10 | 12 | 3 | 11 |

By selecting and exciting different pairs of transmitting coils in sequence or in combination, multiple measurement data can be obtained for image reconstruction.

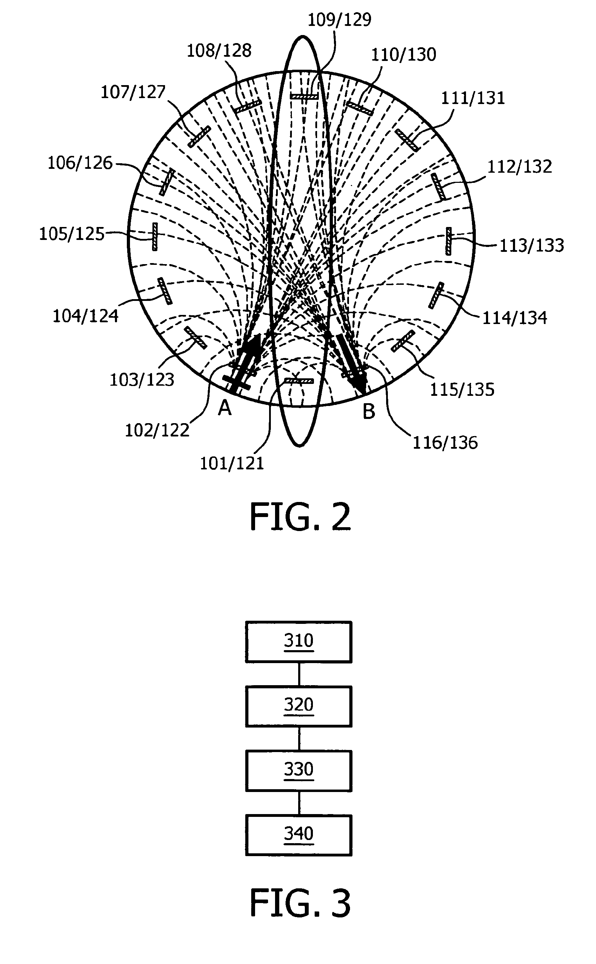

Fig.2 shows a schematic view of a primary magnetic field in accordance with the invention.

Referring to Fig.2, the coil arrangement comprises 16 pairs of transmitting and measuring coils, which are arranged in an annular array and situated around the inner wall of a tubular container 100. In Fig.2, the numerical X denotes the transmitting coil and Y denotes the measurement coil in an X/Y pair.

When the transmitting coils 102 and 116 are simultaneously excited by providing alternating currents of the same magnitude and opposite direction, the transmitting coils 102 and 116 generate magnetic fields having opposite field directions with respect to the normal to the tomography wall. The curves shown by the dotted lines indicate the primary flux distribution, the arrow heads A and B indicate the directions of the flux, and the black ellipsoid indicates areas where the resulting primary flux vanishes.

From Fig. 2, it is observed that the measurement coil 121 is next to and between the transmitting coils 102 and 116, while the measurement coil 129 is farthest away from the transmitting coils 102 and 116. However, according to the excitation scheme provided by the invention, the resulting flux, e.g., the primary magnetic field, at the location of the measurement coils 121 and 129 is theoretically zero for a perfectly symmetric coil arrangement and medium. In practice, it may be larger than zero, but not as large as when one coil is used for excitation in a conventional magnetic induction tomography device, resulting in reduced requirements for the dynamic range of the measurement coils.

Fig.3 is a flowchart of the method in accordance with the invention.

According to the invention, the method comprises a step 310 of selecting and exciting a first pair of transmitting coils 102, 116 among the plurality of transmitting coils 101,102, 103, ...,116, a step 320 of generating a primary magnetic field to be applied to the object of interest by the first pair of transmitting coils 102, 116, and a step 330 of measuring electrical signals induced by a secondary magnetic field for image reconstruction by at least one measurement coil 121, 129, the secondary magnetic field being generated by the object of interest in response to the primary magnetic field.

In an embodiment, the method further comprises a step 340 of selecting and exciting a second pair of transmitting coils 103, 115 among the plurality of transmitting coils, wherein the second pair of transmitting coils is selected and excited in such a way that the primary magnetic field generated by the second pair of transmitting coils is minimized at the location of the at least one first measurement coil 121, 129. The selection can be advantageously implemented by means of a computer program.

In an embodiment, the first or second pair of transmitting coils is excited by simultaneously providing each coil with alternating current of equal magnitude and opposite direction. In this way, the primary magnetic field generated by the first and/or second pair of transmitting coils is zero in theory at the location of the at least one measurement coil.

In an embodiment, the method further comprises a step of arranging the plurality of transmitting coils and the plurality of measurement coils in an annular array that enables easy selection of transmitting coils and measurement coils because of the symmetry of the coil arrangement.

It is advantageous that the plurality of transmitting coils and the plurality of measurement coils are arranged in pairs and situated around the inner wall of a tubular container 100.

It should be noted that the above-mentioned embodiments illustrate rather than limit the invention and that those skilled in the art will be able to design alternative embodiments without departing from the scope of the appended claims. In the claims, any reference signs placed between parentheses shall not be construed as limiting the claim. The word "comprising" does not exclude the presence of elements or steps not listed in a claim or in the description. The word "a" or "an" preceding an element does not exclude the presence of a plurality of such elements. In the apparatus claims enumerating several units, several of these units can be embodied by one and the same item of hardware or software. The usage of the words first, second and third, et cetera, does not indicate any ordering. These words are to be interpreted as names.

## Claims

1. A device for reconstructing images of an object of interest, comprising:
- a plurality of transmitting coils (102, 103, 115, 116) for generating a primary magnetic field;
- a plurality of measurement coils (121, 122, 129, 136); and
- means (150) for selecting and exciting a first pair of transmitting coils (102, 116) among the plurality of transmitting coils,
wherein the first pair of transmitting coils (102, 116) are selected and excited in a way such that the primary magnetic field generated by the first pair of transmitting coils is minimized at the location of at least one measurement coil (121, 129) among the plurality of measurement coils, **characterised in that** the means (150) is further arranged to select and excite a second pair of transmitting coils (103, 115) among the plurality of transmitting coils, and the second pair of transmitting coils is selected and excited in such a way that the primary magnetic field generated by the second pair of transmitting coils is minimized at the location of the at least one first measurement coil (121,129).

2. A device as claimed in claim 1, wherein the first or second pair of transmitting coils is excited by simultaneously providing each coil with alternating current of equal magnitude and opposite direction.

3. A device as claimed in claim 1, wherein the plurality of transmitting coils and the plurality of measurement coils are arranged in an annular array.

4. A device as claimed in claim 3, wherein the plurality of transmitting coils and the plurality of measurement coils are arranged in pairs and situated around the inner wall of a tubular container (100).

5. A method of reconstructing images of an object of interest comprising the steps of:
- selecting and exciting (310) a first pair of transmitting coils among the plurality of transmitting coils;
- generating (320) a primary magnetic field to be applied to the object of interest by the first pair of transmitting coils;
- measuring (330) electrical signals induced by a secondary magnetic field for image reconstruction by at least one measurement coil, the secondary magnetic field being generated by the object of interest in response to the primary magnetic field, **characterised by** the first pair of transmitting coils being selected and excited in a way such that the primary magnetic field generated by the first pair of transmitting coils is minimized at the location of the at least one measurement coil; and -selecting and exciting (340) a second pair of transmitting coils among the plurality of transmitting coils, wherein the second pair of transmitting coils is selected and excited in such a way that the primary magnetic field generated by the second pair of transmitting coils is minimized at the location of the at least one first measurement coil.

6. A method as claimed in claim 5, wherein the first or second pair of transmitting coils is excited by simultaneously providing each coil with alternating current of equal magnitude and opposite direction.

7. A method as claimed in claim 6, further comprising a step of arranging the plurality of transmitting coils and the plurality of measurement coils in an annular array.

8. A device as claimed in claim 7, further comprising a step of arranging the plurality of transmitting coils and the plurality of measurement coils in pairs and around the inner wall of a tubular container.

## Patentansprüche

1. Vorrichtung zum Rekonstruieren von Bildern eines interessierenden Objekts, wobei die Vorrichtung Folgendes umfasst:
- eine Vielzahl von Sendespulen (102, 103, 115, 116) zum Erzeugen eines primären Magnetfelds;
- eine Vielzahl von Messspulen (121, 122, 129, 136); und
- Mittel (150) zum Auswählen und Anregen eines ersten Sendespulenpaares (102, 116) unter der Vielzahl von Sendespulen,
wobei das erste Sendespulenpaar (102, 116) auf derartige Weise ausgewählt und angeregt wird, dass das durch das erste Sendespulenpaar erzeugte primäre Magnetfeld an dem Ort von mindestens einer Messspule (121, 129) unter der Vielzahl von Messspulen minimiert wird, **dadurch gekennzeichnet, dass** die Mittel (150) weiterhin vorgesehen sind, um ein zweites Sendespulenpaar (103, 115) unter der Vielzahl von Sendespulen auszuwählen und anzuregen, und dass das zweite Sendespulenpaar auf derartige Weise ausgewählt und angeregt wird, dass das durch das zweite Sendespulenpaar erzeugte primäre Magnetfeld an dem Ort der mindestens einen ersten Messspule (121, 129) minimiert wird.

2. Vorrichtung nach Anspruch 1, wobei das erste oder das zweite Sendespulenpaar angeregt wird, indem jeder Spule gleichzeitig Wechselstrom von gleicher Magnitude und entgegengesetzter Richtung zugeführt wird.

3. Vorrichtung nach Anspruch 1, wobei die Vielzahl von Sendespulen und die Vielzahl von Messspulen in einem ringförmigen Array angeordnet sind.

4. Vorrichtung nach Anspruch 3, wobei die Vielzahl von Sendespulen und die Vielzahl von Messspulen in Paaren angeordnet sind und um die Innenwand eines röhrenförmigen Behälters (100) herum positioniert sind.

5. Verfahren des Rekonstruierens von Bildern eines interessierenden Objekts, wobei das Verfahren die folgenden Schritte umfasst:
- Auswählen und Anregen (310) eines ersten Sendespulenpaares unter der Vielzahl von Sendespulen;
- Erzeugen (320) eines an das interessierende Objekt anzulegenden primären Magnetfelds durch das erste Sendespulenpaar;
- Messen (330) von durch ein sekundäres Magnetfeld induzierten elektrischen Signalen zur Bildrekonstruktion durch mindestens eine Messspule, wobei das sekundäre Magnetfeld durch das interessierende Objekt in Reaktion auf das primäre Magnetfeld erzeugt wird, **dadurch gekennzeichnet, dass** das erste Sendespulenpaar auf derartige Weise ausgewählt und angeregt wird, dass das durch das erste Sendespulenpaar erzeugte primäre Magnetfeld an dem Ort der mindestens einer Messspule minimiert wird; und
- Auswählen und Anregen (340) eines zweiten Sendespulenpaares unter der Vielzahl von Sendespulen, wobei das zweite Sendespulenpaar auf derartige Weise ausgewählt und angeregt wird, dass das durch das zweite Sendespulenpaar erzeugte primäre Magnetfeld an dem Ort der mindestens einen ersten Messspule minimiert wird.

6. Verfahren nach Anspruch 5, wobei das erste oder das zweite Sendespulenpaar angeregt wird, indem jeder Spule gleichzeitig Wechselstrom von gleicher Magnitude und entgegengesetzter Richtung zugeführt wird.

7. Verfahren nach Anspruch 6, das weiterhin einen Schritt des Anordnens der Vielzahl von Sendespulen und der Vielzahl von Messspulen in einem ringförmigen Array umfasst.

8. Verfahren nach Anspruch 7, das weiterhin einen Schritt des Anordnens der Vielzahl von Sendespulen und die Vielzahl von Messspulen in Paaren und um die Innenwand eines röhrenförmigen Behälters herum umfasst.

## Revendications

1. Dispositif de reconstruction d'images d'un objet d'intérêt, comprenant :
- une pluralité de bobines de transmission (102, 103, 115, 116) pour générer un champ magnétique primaire ;
- une pluralité de bobines de mesure (121, 122, 129, 136) ; et
- un moyen (150) pour sélectionner et exciter une première paire de bobines de transmission (102, 116) parmi la pluralité de bobines de transmission,
dans lequel la première paire de bobines de transmission (102, 116) est sélectionnée et excitée de telle manière que le champ magnétique primaire généré par la première paire de bobines de transmission soit minimisé à l'emplacement d'au moins une bobine de mesure (121, 129) parmi la pluralité de bobines de mesure, **caractérisé en ce que** le moyen (150) est en outre agencé pour sélectionner et exciter une deuxième paire de bobines de transmission (103, 115) parmi la pluralité de bobines de transmission, et la deuxième paire de bobines de transmission est sélectionnée et excitée de telle manière que le champ magnétique primaire généré par la deuxième paire de bobines de transmission soit minimisé à l'emplacement de l'au moins une première bobine de mesure (121, 129).

2. Dispositif selon la revendication 1, dans lequel la première ou deuxième paire de bobines de transmission est excitée en fournissant simultanément à chaque bobine un courant alternatif de grandeur égale et de direction opposée.

3. Dispositif selon la revendication 1, dans lequel la pluralité de bobines de transmission et la pluralité de bobines de mesure sont agencées dans un réseau annulaire.

4. Dispositif selon la revendication 3, dans lequel la pluralité de bobines de transmission et la pluralité de bobines de mesure sont agencées en paires et situées autour de la paroi intérieure d'un conteneur tubulaire (100).

5. Procédé de reconstruction d'images d'un objet d'intérêt comprenant les étapes de :
- la sélection et l'excitation (310) d'une première paire de bobines de transmission parmi la pluralité de bobines de transmission ;
- la génération (320) d'un champ magnétique primaire à appliquer à l'objet d'intérêt par la première paire de bobines de transmission ;
- la mesure (330) de signaux électriques induits par un champ magnétique secondaire pour une reconstruction d'images par au moins une bobine de mesure, le champ magnétique secondaire étant généré par l'objet d'intérêt en réponse au champ magnétique primaire, **caractérisé en ce que** la première paire de bobines de transmission est sélectionnée et excitée de telle manière que le champ magnétique primaire généré par la première paire de bobines de transmission soit minimisé à l'emplacement d'au moins une bobine de mesure ; et
- la sélection et l'excitation (340) d'une deuxième paire de bobines de transmission parmi la pluralité de bobines de transmission, dans lequel la deuxième paire de bobines de transmission est sélectionnée et excitée de telle manière que le champ magnétique primaire généré par la deuxième paire de bobines de transmission soit minimisé à l'emplacement de l'au moins une première bobine de mesure.

6. Procédé selon la revendication 5, dans lequel la première ou deuxième paire de bobines de transmission est excitée en fournissant simultanément à chaque bobine un courant alternatif de grandeur égale et de direction opposée.

7. Procédé selon la revendication 6, comprenant en outre une étape d'agencement de la pluralité de bobines de transmission et la pluralité de bobines de mesure dans un réseau annulaire.

8. Procédé selon la revendication 7, comprenant en outre une étape d'agencement de la pluralité de bobines de transmission et la pluralité de bobines de mesure en paires et autour de la paroi intérieure d'un conteneur tubulaire.
